# EUROPEAN PATENT SPECIFICATION

(11) **EP 2 245 354 B1**
(45) Date of publication and mention of the grant of the patent: **13.06.2012**
(21) Application number: 09706037.0
(22) Date of filing: 28.01.2009
(51) Int. Cl.: F16L 37/098

(54) **QUICK CONNECT/DISCONNECT COUPLING ASSEMBLIES**
KUPPLUNGSANORDNUNGEN MIT SCHNELLVERBINDUNG/SCHNELLLÖSUNG
ENSEMBLES D'ACCOUPLEMENT A DECONNEXION/CONNEXION RAPIDE

(30) Priority: 28.01.2008 US 24044; 27.03.2008 US 40045
(43) Date of publication of application: 03.11.2010
(73) Proprietor: COLDER PRODUCTS COMPANY, St. Paul, MN 55114 (US)
(72) Inventor: WILLIAMS, Randall, Scott, Minneapolis MN 55410 (US); GERST, Patrick, Thomas, Oakdale MN 55128 (US); WILHELM, Grant, Armin, Plymouth MN 55447 (US); HANSON, James, Mark, Woodbury MN 55125 (US)
(74) Representative: Quinterno, Giuseppe
(86) International application number: PCT/US2009/032226
(87) International publication number: WO 2009/097327

(56) References cited:
- EP-A- 0 982 525
- WO-A1-2004/071568
- GB-A- 2 323 418
- US-A- 5 964 485

## Description

The present invention relates to a coupling assemby of the kind defined in the preamble of claim 1.

A coupling device of this kind and a molding method are disclosed in GB-2 323 418 A and US-5 964 485 A.

An object of the present invention is to provide an improved coupling assembly of the above-defined kind.

This object is achieved according to the invention by the coupling assembly defined in claim 1.

Reference is now made to the accompanying drawings, which are not necessarily drawn to scale.
Figure 1 is a schematic view of an example system including a quick connect / disconnect coupling assembly in an uncoupled state.
Figure 2 is a perspective view of an example connector body.
Figure 3 is a side cross-sectional view of the connector body of Figure 2.
Figure 4 is a top cross-sectional view of the connector body of Figure 2.
Figure 5 is a perspective view of an example connector insert.
Figure 6 is a cross-sectional view of the connector insert of Figure 5.
Figure 7 is a perspective view of a quick connect / disconnect coupling assembly including the connector body of Figure 2 and the connector insert of Figure 5 in a coupled state.
Figure 8 is a side cross-sectional view of the coupling assembly of Figure 7.
Figure 9 is a top cross-sectional view of the coupling assembly of Figure 7.
Figure 10 is flowchart showing an example method of using a quick connect / disconnect coupling assembly.
Figure 11 is an example mold for forming the connector body of Figure 2.
Figure 12 is another view of the example mold shown in Figure 11.
Figure 13 is a top cross-sectional view of an alternative embodiment of the connector body.
Figure 14 is a side cross-sectional view of the connector body of Figure 13.
Figure 15 is a top cross-sectional view of another alternative embodiment of the connector body.
Figure 16 is a side cross-sectional view of the connector body of Figure 15.
Figure 17 is a flowchart showing another example method of manufacturing a quick connect / disconnect coupling assembly.

The present disclosure relates generally to quick connect /disconnect coupling assemblies. The mated coupling assembly provides a fluid tight, smooth and continuous fluid flow path that minimizes dead volume space.

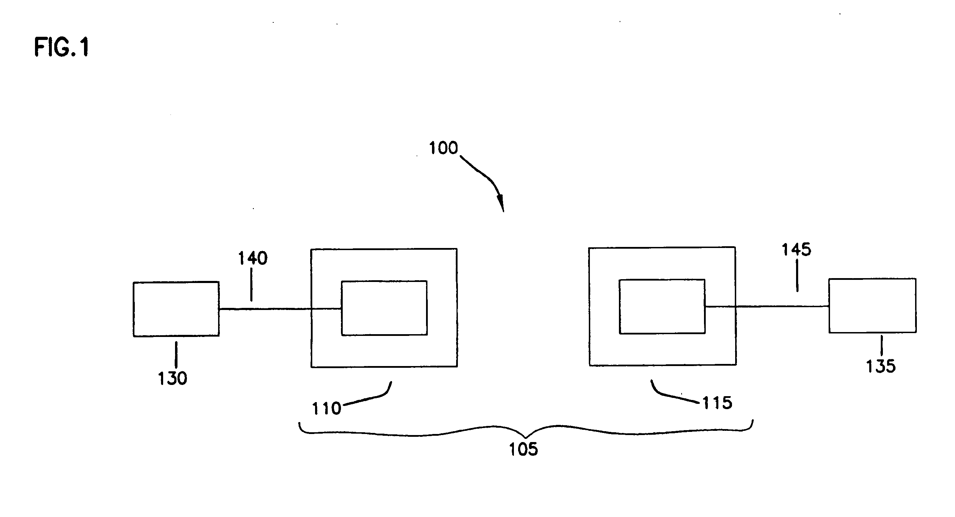

Referring now to Figure 1, an example system 100 including a coupling assembly 105 is shown. Additionally, a fluid source receptacle 130, a fluid termination receptacle 135, a conduit 140, and a conduit 145 are shown in system 100. The coupling assembly 105 includes a female coupling 110 and a male coupling 115.

In the example system 100, one end of the conduit 140 is connected to the fluid source receptacle 130, and the other end of the conduit 140 is connected to the female coupling 110 of the coupling assembly 105. Further, one end of the conduit 145 is connected to the fluid termination receptacle 135, and the other end of conduit 145 is connected to the male coupling 115 of the coupling assembly 105. The female coupling 110 and the male coupling 115 are mated to form a continuous fluid flow path to allow the flow of fluid therethrough from the fluid source receptacle 130 to the fluid termination receptacle 135.

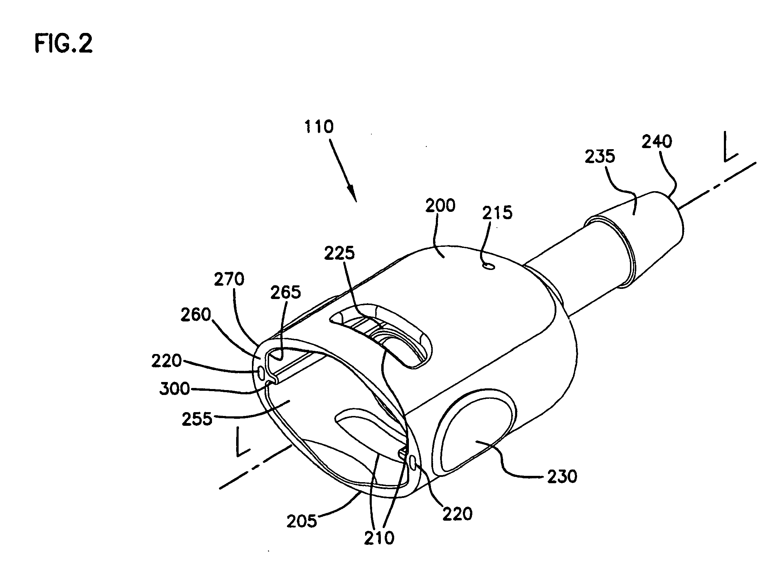

Referring now to Figures 2-4, the female coupling 110 is shown. The female coupling 110 includes a first opening aperture 205, a retainment aperture 210, and a lead-in receptacle 225 to facilitate the mating of the female coupling 110 with complementary structures on the male coupling 115.

In the embodiment shown, the first opening aperture 205 is normally elliptical in shape when in a resting state. The first opening aperture 205 is a mechanically deformable aperture including a lip member 260 defined by an inner periphery 265 and an outer periphery 270, and a protruding rib 300. The first opening aperture 205 also includes a smooth beveled lead-in surface 305 generally extending around the inner periphery 265 of the first opening aperture 205 to reduce the force required for insertion of male coupling 115. As described further below, the first opening aperture 205 is deformable when force is applied to fitted thumb pads 230.

In the embodiment shown, the retainment aperture 210 of the female coupling 110 is oblong and sized to receive complementary coupling features on the male coupling 115. Additionally, immediately adjacent to the retainment aperture 210 is a collar tab 355 used to accept a flange member collar 625 on the male coupling 110.

In the embodiment, the female coupling 110 includes the lead-in receptacle 225 defined inside an inner housing 255 of the female coupling body. The lead-in receptacle 225 is a cylindrical tube protruding from a base 310 of the body inner housing 255 of the female coupling 110 to a first leading edge 325 of the retainment aperture 210.

The lead-in receptacle 225 further includes a front surface 320 defined by a beveled lead-in surface 315. The beveled lead-in surface 315 generally extends around the inner periphery of the front surface 320 to provide entry of complementary coupling features on the male coupling 115, as described further below.

The protruding rib 300 generally extends along a longitudinal axis L from the lip member 260 along the inner housing 255 therewith. The protruding rib 300 facilitates mating of the female coupling 110 with the male coupling 115, as described further below.

In the embodiment, the female coupling 110 includes an end opening 240 opposite of the coupling mechanisms. The female coupling end opening 240 is connected to a female coupling fluid channel 335. Radially adjacent to the end opening 240 is a hose barb 235, which generally flares out as the barb 235 extends inwardly towards body inner housing 255 along longitudinal axis L until terminating at a right angle 350 with respect to longitudinal axis L. The hose barb 235 facilitates secure connections to conduits (e.g., conduit 140 describe above) running to various equipment or other applications.

In the embodiment shown, the female coupling 110 includes a deformable fluid seal 330 comprising a primary sealing surface 405 and a secondary sealing surface 410 to provide fluid tight seals of a mated coupling assembly, as described further below. A distance A is defined as the distance between a primary stop surface 365 and a fluid seal first leading edge 360.

The female coupling 110 also includes a circular gate aperture 215, an elliptical gate aperture 220 on each side of the lip member 260 , and a circular vent 380 on the female coupling body 200. The gates 215, 220 and the vent 380 are utilized during an injection molding process (described below) to facilitate the formation of the fluid seal 330 and the thumb pad 230.

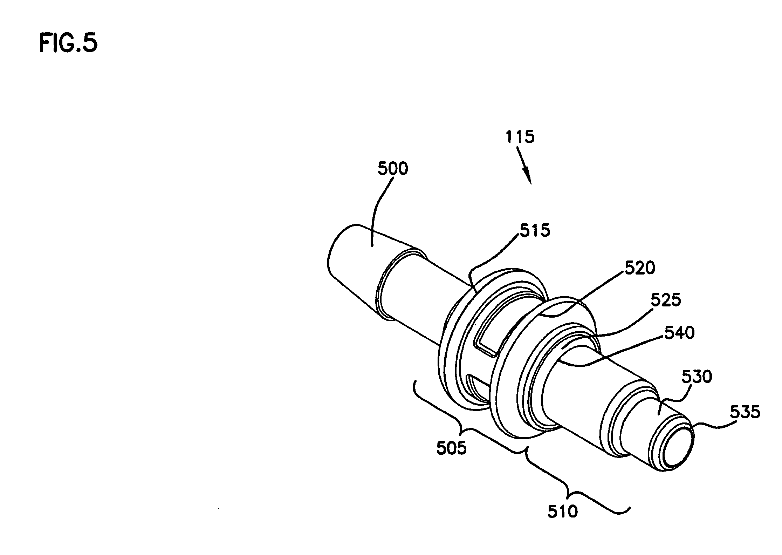

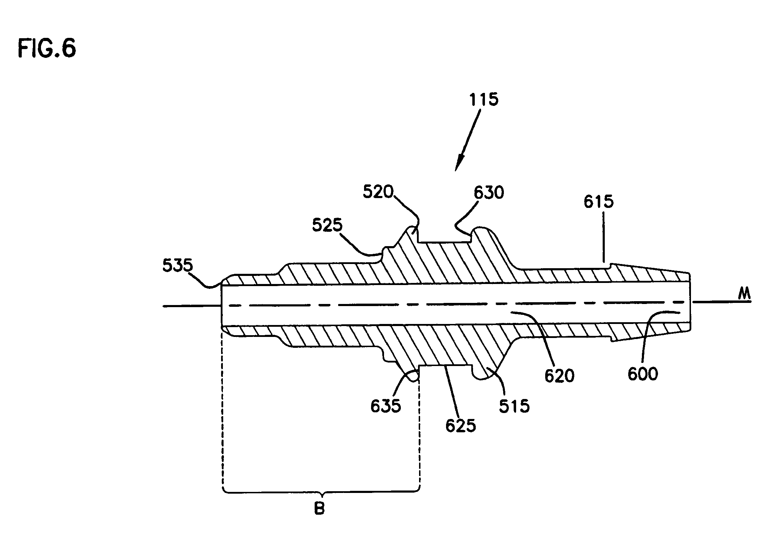

Referring now to Figures 5 and 6, the male coupling 115 is shown. The male coupling 115 includes a hose barb 500, a flange member 505, and an insert member 510 to facilitate the mating of the male coupling 115 with complementary structures on the female coupling 110. In the example shown, the male coupling 115 is axially symmetric.

The male coupling 115 includes an outer clip flange 515 generally flaring out and extending inwardly towards the insert member 510 along a longitudinal axis M until terminating at a right angle at a portion 630 with respect to longitudinal axis M. Additionally, the male coupling 115 also includes an inner clip flange 520 generally flaring out and extending inwardly towards the hose barb 500 along longitudinal axis M until terminating at an inner clip flange primary edge 635. Further, the male coupling 115 includes a flange member collar 625 generally flanked by the outer clip flange 515 and the inner clip flange 520.

Additionally, the male coupling 115 also includes a hard stop lip member 525 generally extending inwardly towards the inner clip flange 520 along longitudinal axis M. The hard stop lip member 525 includes a first leading edge 540. The flange member 505 includes the flange member collar 625, the outer clip flange 515, the inner clip flange 520, and the hard stop lip member 525.

In example embodiments, the male coupling 115 also includes a member 510 comprising a primary sealing surface 535 and a secondary sealing surface 530 to facilitate sealing with complementary features on the fluid seal 330, as described further below. In addition, a distance B (see Figure 6) is defined as the distance between the primary sealing surface 535 and the inner clip flange primary edge 635 on the male coupling 115. In example embodiments, the inner diameters of the primary sealing surface 405 and the primary sealing surface 535 are sized to approximate the diameters of the fluid channels 335, 620 to minimize dead volume.

The male coupling 115 includes an end opening 600 opposite of the insert member 510. The end opening 600 is connected to a male coupling fluid channel 620. Radially adjacent to the end opening 600 is a hose barb 500, generally flaring out extending inwardly towards flange member 505 along longitudinal axis M until terminating at right angle 615 with respect to longitudinal axis M. The hose barb 500 facilitates secure connections to conduits (e.g., conduit 145) running to various equipment or other applications.

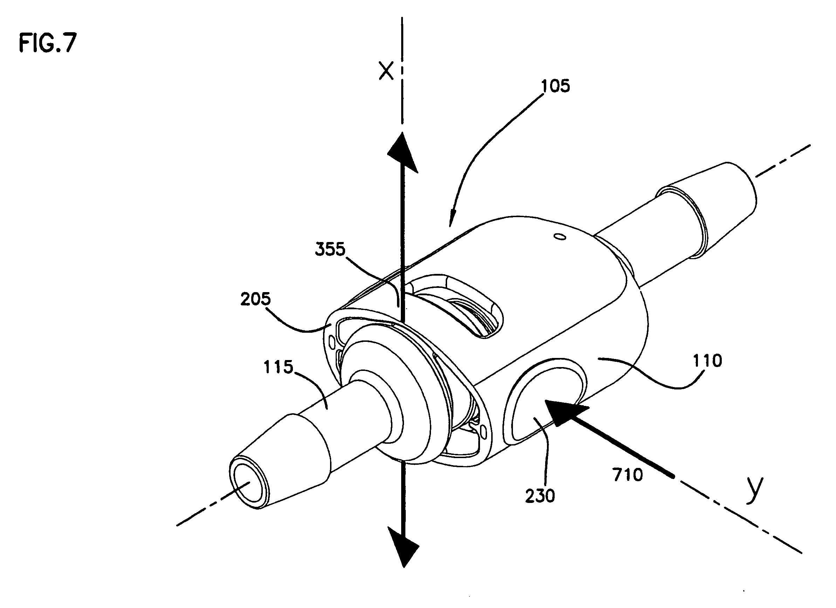

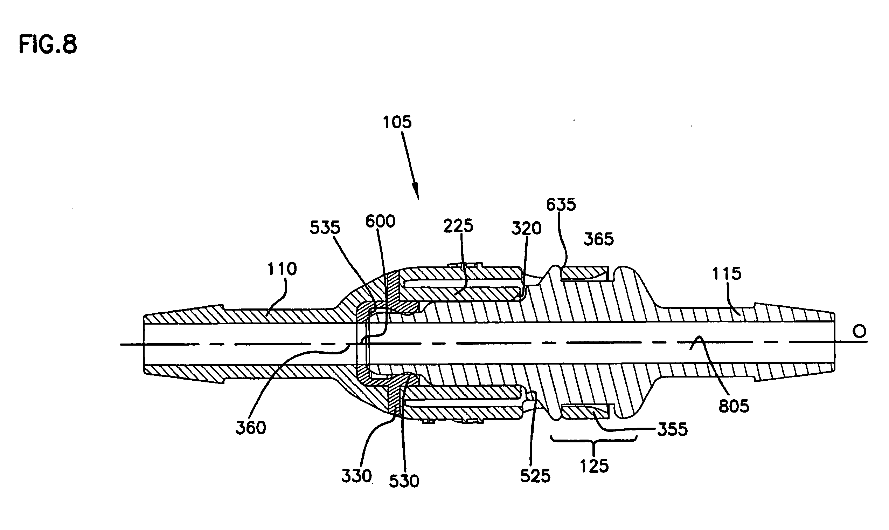

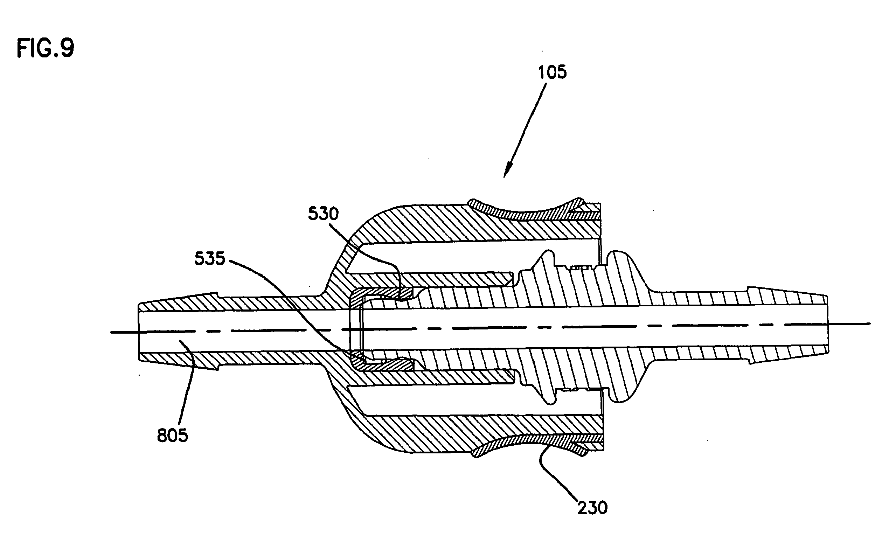

Referring now to Figures 7-9, the coupling assembly 105 is shown including the male coupling 115 and female coupling 110 in a coupled state. Generally, the female coupling 110 and male coupling 115 of the coupling assembly 105 are mated via a push-to-connect process, thereby forming a fluid tight pressure seal. As note above, the male coupling 115 is axially symmetric and can be positioned in any orientation when coupled to the female coupling 110.

In the embodiment shown, the male coupling is orientated such that the insert member 510 of the male coupling 115 is pointing towards and inserted into the first opening aperture 255 of the female coupling 110. Then, an initial force along the longitudinal axis O is required for insertion of the insert member 510 into the first opening aperture 255. The insert member 510 is received by the lead-in receptacle 225 on the female coupling 110 and forward motion proceeds until the leading edge of the male coupling inner clip flange 520 interacts with the first opening aperture 255. Additional force is required to allow the inner clip flange 520 to mechanically deform the first opening aperture 255 along a direction X.

Upon deformation of the first opening aperture 255, the collar tab 355 of the male coupling 115 is allowed to engage with the flange member collar 125 of the female coupling 110. The distance A between the primary stop surface 365 and the fluid seal first leading edge 360 is smaller than the distance B between the primary sealing surface 535 and the inner clip flange primary edge 635. Forward motion proceeds until the male coupling primary sealing surface 535 compresses against the female coupling primary sealing surface 405 resulting in a fluid tight pressure seal due to the difference between distances A and B.

The user then releases the male coupling 115 and the resultant energy stored in the deformable fluid seal 330 repels the male coupling 115 in the opposite direction on longitudinal axis O until the inner clip flange primary edge 635 interacts with the female coupling retainment aperture 255. A fluid tight pressure seal fitting is maintained due to contact between the inner clip flange primary edge 635 and the primary stop surface 365.

In the coupled state, a continuous fluid flow path is created by seamless connection of female coupling fluid flow channel 335 and male coupling fluid flow 620 channel to form a mated coupling assembly channel 805.

The female and male couplings 110, 115 of the coupling assembly 105 are disengaged by applying force along a direction Y to the thumb pads 230 to mechanically deform the first opening aperture 205 on the female coupling 110 in a direction X. Upon deformation of the first opening aperture 255, the collar tab 355 of the male coupling 115 is disengaged with the flange member collar 125 of the female coupling 110 and the male coupling insert member 510 is removed from the inner housing.

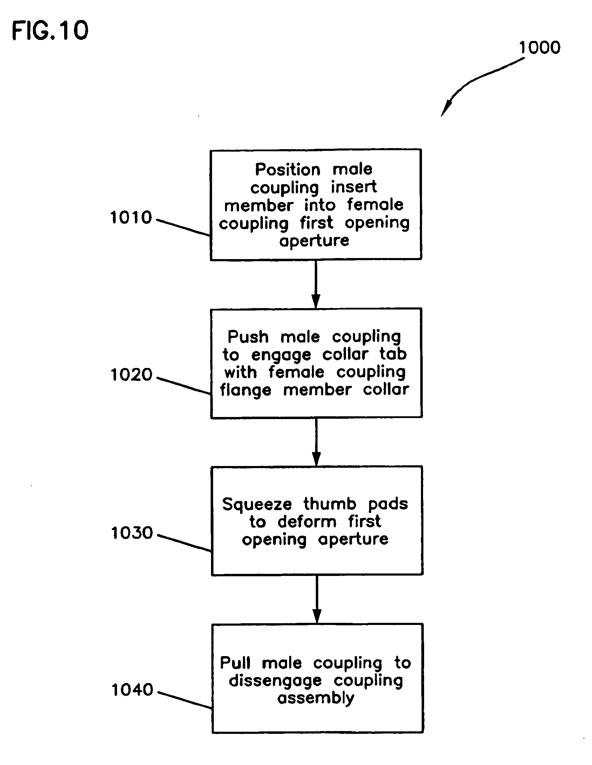

Referring now to Figure 10, an example method 1000 is shown to engage and disengage the male coupling and the female coupling. Initially, at operation 1010, the user positions the male coupling insert member into the first opening aperture of the female coupling. Next, at operation 1020, the male coupling is pushed until the collar tab of the male coupling is allowed to engage with the flange member collar of the female coupling. At this point, the male coupling is connected to the female coupling and a fluid tight passage is formed therebetween.

To decouple the male coupling from the female coupling, the processes continues at operation 1030, where the user applies force to the fitted thumb pads on the female coupling. This causes the first opening aperture to be mechanically deformed thereby disengaging flange member from retainment aperture. Next, at operation 1040 the male coupling is pulled from female coupling while maintaining pressure on the fitted thumb pads.

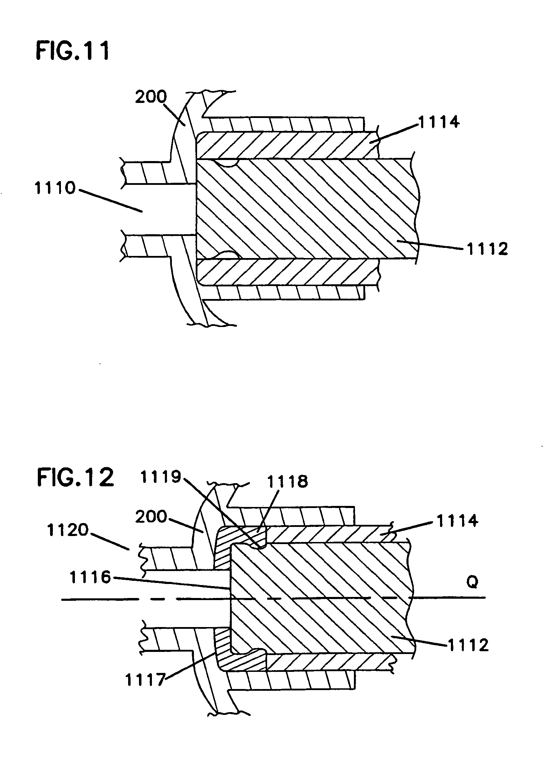

Referring now to Figures 11-13, an example system and method for the manufacture the female coupling 110 is disclosed. Generally, in the example shown, the injection overmolding process includes a two step or "two-shot" procedure.

Referring now to Figures 11 and 12, portions of an example mold 1110 for forming portions of the female coupling 110 are shown. The mold 1110 includes an inner core 1112 and an outer core 1114. This mold 1110 is an example only. Other molds and molding techniques can be used.

As shown in Figure 11, during a first shot of the two-shot process, the female coupling body 200 is formed by injecting thermoplastic material into the mold 1110. (Note: Only a portion of the mold 1110 and resulting female coupling body 200 are shown in Figure 11).

Now referring now to Figure 12, during the second step of the overmolding process or "second shot," the fluid seal 330 and the thumb pads 230 of the female coupling 110 are formed using a softer thermoplastic elastomer material. To form the fluid seal 330, both the inner core 1112 and the outer core 1114 are moved axially in a direction Q. Specifically, the inner core 1112 is moved so that an end surface 1116 is positioned away from a surface 1117 of the female coupling body 200. The outer core 1114 is moved further axially in the direction Q such that a cavity 1118 is formed by an end surface 1117 which includes indentations 1119 formed in the inner core 1112. With the cores 1112, 1114 in this position, the second shot of the softer thermoplastic material is injected to form the fluid seal 330. Although not shown, the thumb pads 230 can be formed using a similar process during the second shot.

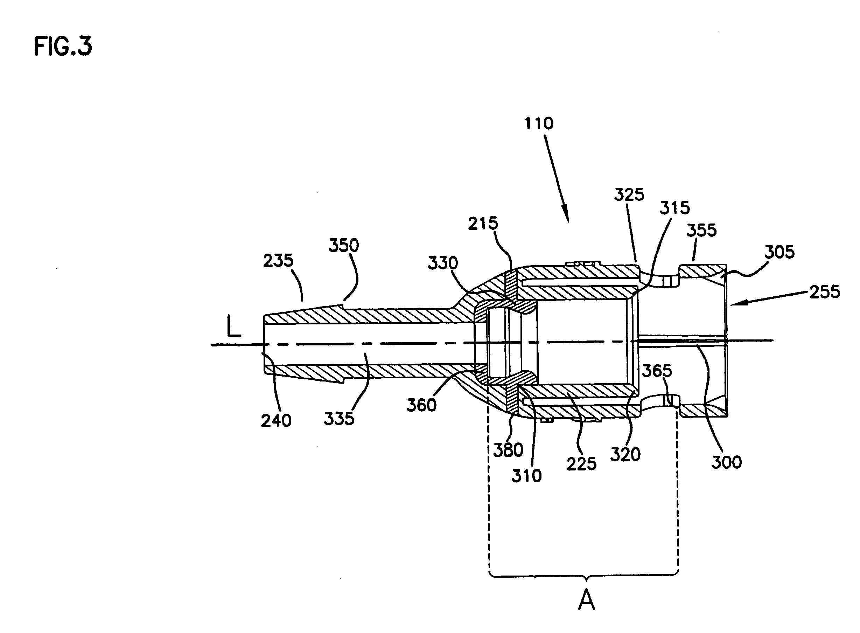

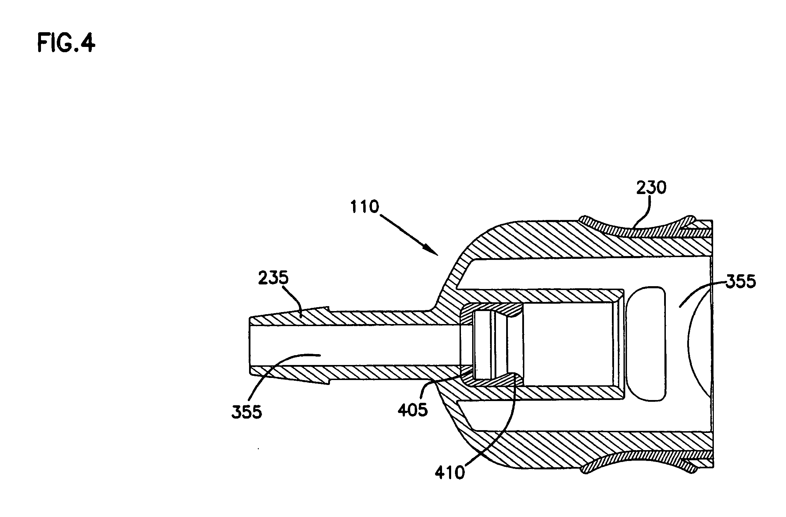

In the examples shown, the female coupling body 200 includes the circular gate aperture 215 and the circular vent 380 used for injection of the thermoplastic material to define the fluid seal 330 (see Figure 3), and an elliptical gate aperture 220 for thermoplastic material injection to define the thumb pad 230, (see Figure 4).

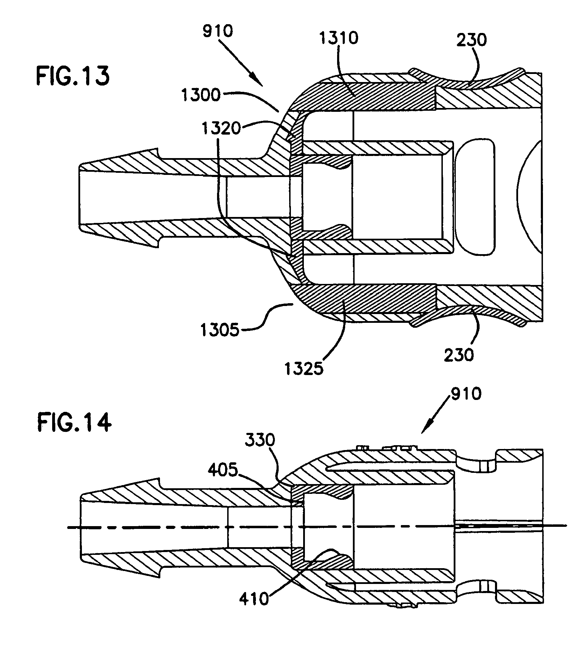

Referring now to Figures 13 and 14, in an alternative embodiment a female coupling 910 is molded using only a single gate aperture 1300 and a single vent aperture 1305. The fluid seal 330 and the thumb pads 230 of the female coupling 910 are formed using a soft thermoplastic elastomer material in a single second shot injection step. The second shot of the softer thermoplastic material is injected into the gate aperture 1300. The material is forced along a first duct 1310 to form one of the thumb pads 230. The material is also forced along a second duct 1320 to form the fluid seal 330. Additionally, the second shot thermoplastic material is forced into a third duct 1325 to form the second thumb pad 230. Venting of the mold cavity is provided via the vent aperture 1305. In example embodiments, the ducts 1310, 1320, 1325 can be formed at right angles with respect to one another, or at obtuse or acute angles with respect to one another.

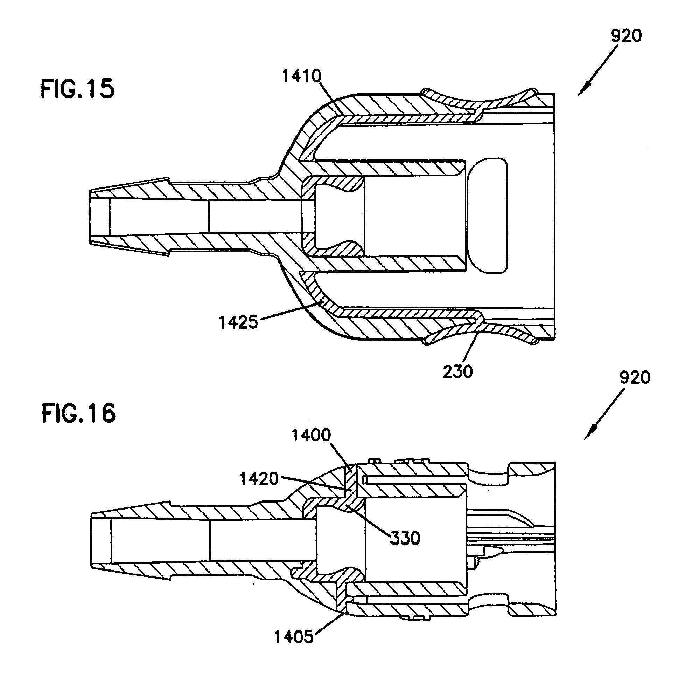

Referring now to Figures 15 and 16, in an alternative embodiment a female coupling 920 is molded using a single gate aperture 1400 and a single vent aperture 1405. The fluid seal 330 and the thumb pads 230 of the female coupling 920 are formed using a soft thermoplastic elastomer material in a single second shot injection step. The second shot of the softer thermoplastic material is injected into the gate aperture 1400. The material is forced along a first duct 1410 to form one of the thumb pads 230. The material is also forced along a second duct 1420 to form the fluid seal 330. Additionally, the second shot thermoplastic material is forced into a third duct 1425 to form the second thumb pad 230. Venting of the mold cavity is provided via the vent aperture 1405. In example embodiments, the ducts 1410, 1420, 1425 can be formed at right angles with respect to one another, or at obtuse or acute angles with respect to one another.

In example embodiments, the thermoplastic used to form the overmolded fluid seal and the thumb pads is a thermoplastic elastomer ("TPE") or a thermoplastic vulcanizate ("TPV"). In one example, TPV is formed using a resin sold under the trademark SANTOPRENE™ by Advanced Elastomer Systems, LP of Akron, Ohio. Other materials, such as KRATON® and/or VERSALLOY® manufactured by GLS Corporation of McHenry, Illinois, or TEKBOND® manufactured by Teknor Apex Company of Pawtucket, Rhode Island, can also be used.

In example embodiments, a lubricant can be added to the thermoplastic elastomer (e.g., TPE) to reduce friction during coupling and uncoupling and to promote increased life of the seals. However, in some applications, such as those in the medical industry, the addition of an external lubricant is undesirable. In such applications, an internal friction reducing component can be added to the TPE. One such friction reducing component is MedGLIDE manufactured by Clariant Corporation of Charlotte, North Carolina. Various ratios of the friction reducing component can be added to the TPE. In some examples, the friction reducing component makes up between 0.5 to 5 percent, more preferably between 1 to 4 percent, and even more preferably 2 or 4 percent of the total TPE. The MedGLIDE can be a reactive additive that is added during the compounding of the TPE or other thermoplastic.

Other types of friction reducing components in varying amounts can also be used. For example, in another embodiment, a friction reducing additive such as FLUOROGUARD® Polymer Additive manufactured by DUPONT™ can be used. In yet other examples, the friction reducing component can be added to the thermoplastic parts that interface with the thermoplastic elastomer, rather than or in addition to the thermoplastic elastomer itself. For example, in an alternative embodiment, the friction reducing component (e.g., MedGLIDE or FLUOROGUARD) is added to the male coupling to reduce the friction created between the portions of the male coupling that contact the thermoplastic elastomer seals on the female coupling during coupling and uncoupling. Besides the benefit of reduced friction, such configurations can also have other advantages as well. For example, the friction reducing components can allow for a better audible "click" that is created when the male coupling is fully inserted into the female coupling, thereby providing feedback to the user that a full connection has been achieved.

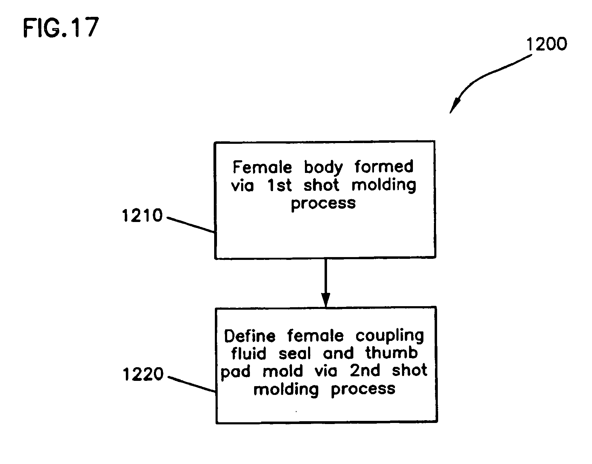

Referring now to Figure 17, an example method 1200 for forming the female fluid coupling 115 is shown. Initially, at operation 1210, the female coupling body is formed during the first shot of the two shot molding process. Next, at operation 1220, the female coupling fluid seal and thumb pads are formed during the second shot of the two shot molding process.

The preceding embodiments are intended to illustrate without limitation the utility and scope of the present disclosure. Those skilled in the art will readily recognize various modifications and changes that may be made to the embodiments described above without departing from the scope of the disclosure.

## Claims

1. A quick connect/disconnect coupling assembly (105), including a female coupling (110) and a mating, male coupling (115), the female coupling (110), comprising:
a main body (200) defining a fluid passage (335) therethrough;
a first end (255) defining an opening (205);
a receptacle member (225) positioned within the main body (200) about the fluid passage (335), the receptacle member (225) being sized to receive a portion of said mating coupling (115) that is insertable through the opening (205) in the first end (255); and
a deformable seal member (330) positioned within the receptacle member (225), the seal member (330) including a sealing surface to provide a fluid tight seal with the mating coupling (115), wherein said seal member (330) includes two sealing surfaces (405, 410) comprising a primary sealing surface (405) positioned to engage an end (535) of the mating coupling (115), and a secondary sealing surface (410) to engage a side surface (530) of the mating coupling (115);
the coupling assembly (105) being **characterized in that** said secondary sealing surface (410) is positioned in a perpendicular orientation with respect to the primary sealing surface (405).

2. The coupling assembly (105) of claim 1, wherein:
the opening (205) in the first end (255) of the main body (200) is elliptical in shape when in a resting state;
the opening (205) in the first end (255) is deformable so that the portion of the mating coupling (115) can be inserted into the opening (205); and
the main body (200) of the female coupling (110) further includes a flange member collar (355), the flange member collar (355) being configured to engage the mating coupling (115) when the portion of the mating coupling (115) is inserted into the opening (205).

3. The coupling assembly (105) of claim 2, wherein the first end (255) is deformable to disengage the flange member collar (355) from the mating coupling (115) to allow the mating coupling (115) to be removed from the opening (205) of the coupling (110).

4. The coupling assembly (105) (110) of claim 3, the female coupling further comprising opposing thumb pads (230) positioned about the main body (200) to allow a user to contact the thumb pads (230) and deform the first end (255).

5. The coupling assembly (105) of claim 1, wherein the primary and secondary sealing surfaces (405, 410) of the seal member (330) are formed during a single overmolding process.

6. The coupling assembly (105) according to any of the preceding claims **characterized by**
a first coupling device (100) according to any of the preceding claims, and
a second coupling device (115) adapted to be inserted through the first opening (205) in the first end (255);
the male coupling (115) including:
a second main body (510) including an outer surface (530) and defining a second fluid passage (620);
a first end (535) including an end surface surrounding a second opening; and
a first flange member (520) extending from the outer surface of the second main body (510);
wherein the end surface of the first end (535) of the male coupling (115) engages the primary sealing surface (405), and the secondary sealing surface (410) engages the outer surface (530) of the main body (510) of the male coupling (115) when the male coupling (115) is coupled to the female coupling (110).

7. The coupling assembly of claim 6, wherein the first opening (205) in the first end (255) of the female coupling (110) is deformable as the portion of the male coupling (115) is inserted into the first opening (205) so that the portion of the male coupling (115) can be inserted into the receptacle member (225).

8. The coupling assembly (105) of claim 7, wherein the first main body (200) of the female coupling (110) further includes a flange member collar (355), the flange member collar (355) being configured to be positioned to engage the first flange member (520) extending from the outer surface of the second main body (510) of the male coupling (115) when the portion of the male coupling (115) is inserted into the first opening (205) of the female coupling (110).

9. The coupling assembly (105) of claim 8, wherein the first end (255) of the female coupling (110) is deformable to allow the flange member collar (355) to disengage the first flange member (520) to allow the portion of the male coupling (115) to be removed from the first opening (205) of the female coupling (110).

10. The coupling assembly (105) of claim 4, wherein the thumb pads (230) and the seal member (330) are formed during a single overmolding process.

## Patentansprüche

1. Kupplungsanordnung (105) mit Schnellverbindung/Schnelllösung, welche eine weibliche Kupplung (110) und eine dazupassende männliche Kupplung (115) aufweist, wobei die weibliche Kupplung (110) Folgendes aufweist:
einen Hauptkörper (200), welcher durch diesen hindurch einen Fluiddurchgang (335) definiert;
ein erstes Ende (255), welches eine Öffnung (205) definiert;
ein Aufnahmeglied (225), das innerhalb des Hauptkörpers (200) um den Fluiddurchgang (335) herum angeordnet ist, wobei das Aufnahmeglied (225) derart bemessen ist, dass es einen Abschnitt der dazupassenden Kupplung (115) aufnimmt, welche durch die Öffnung (205) in dem ersten Ende (255) eingeführt werden kann; und
ein verformbares Dichtungsglied (330), welches innerhalb des Aufnahmeglieds (225) angeordnet ist, wobei das Dichtungsglied (330) eine Dichtungsoberfläche aufweist, um eine fluiddichte Abdichtung mit der dazupassenden Kupplung (115) bereitzustellen, wobei das Dichtungsglied (330) zwei Dichtungsoberflächen (405, 410) aufweist, welche eine primäre Dichtungsoberfläche (405), die angeordnet ist, um mit einem Ende (535) der dazupassenden Kupplung (115) in Eingriff zu stehen, und eine sekundäre Dichtungsoberfläche (410), um mit einer Seitenoberfläche (530) der dazupassenden Kupplung (115) in Eingriff zu stehen, umfassen;
wobei die Kupplungsanordnung (105) **dadurch gekennzeichnet ist, dass** die sekundäre Dichtungsoberfläche (410) in einer orthogonalen Ausrichtung in Bezug auf die primäre Dichtungsoberfläche (405) angeordnet ist.

2. Kupplungsanordnung (105) nach Anspruch 1, wobei:
die Öffnung (205) in dem ersten Ende (255) des Hauptkörpers (200) in einem Ruhezustand eine elliptische Gestalt aufweist;
die Öffnung (205) in dem ersten Ende (255) derart verformbar ist, dass der Abschnitt der dazupassenden Kupplung (115) in die Öffnung (205) eingeführt werden kann; und
der Hauptkörper (200) der weiblichen Kupplung (110) ferner einen Flanschgliedhals (355) aufweist, wobei der Flanschgliedhals (355) derart ausgestaltet ist, dass er mit der dazupassenden Kupplung (115) in Eingriff gelangt, wenn der Abschnitt der dazupassenden Kupplung (115) in die Öffnung (205) eingeführt wird.

3. Kupplungsanordnung (105) nach Anspruch 2, wobei das erste Ende (255) verformbar ist, um den Flanschgliedhals (355) mit der dazupassenden Kupplung (115) außer Eingriff zu bringen, um zu ermöglichen, die dazupassende Kupplung (115) aus der Öffnung (205) der Kupplung (110) zu lösen.

4. Kupplungsanordnung (105) nach Anspruch 3, wobei die weibliche Kupplung (110) ferner entgegengesetzt angeordnete Daumenkissen (230) aufweist, welche um den Hauptkörper (200) herum angeordnet sind, um zu ermöglichen, dass ein Benutzer die Daumenkissen (230) berührt und das erste Ende (255) verformt.

5. Kupplungsbaugruppe (105) nach Anspruch 1, wobei die primäre und die sekundäre Dichtungsoberfläche (405, 410) des Dichtungsglieds (330) während eines einzigen Umspritzungsprozesses geformt werden.

6. Kupplungsanordnung (105) nach einem beliebigen der vorhergehenden Ansprüche, **gekennzeichnet durch**:
eine erste Kupplungsvorrichtung (100) nach einem beliebigen der vorhergehenden Ansprüche, und
eine zweite Kupplungsvorrichtung (115), die ausgebildet ist, um **durch** die erste Öffnung (205) in dem ersten Ende (255) eingeführt zu werden;
wobei die männliche Kupplung (115) Folgendes aufweist:
einen zweiten Hauptkörper (510), der eine äußere Oberfläche (530) aufweist und einen zweiten Fluiddurchgang (620) definiert;
ein erstes Ende (535), das eine Endoberfläche umfasst, welche eine zweite Öffnung umgibt; und
ein erstes Flanschglied (520), welches sich von der äußeren Oberfläche des zweiten Hauptkörpers (510) weg erstreckt;
wobei die Endoberfläche des ersten Endes (535) der männlichen Kupplung (115) mit der primären Dichtungsoberfläche (405) in Eingriff gelangt und die sekundäre Dichtungsoberfläche (410) mit der äußeren Oberfläche (530) des Hauptkörpers (510) der männlichen Kupplung (115) in Eingriff gelangt, wenn die männliche Kupplung (115) mit der weiblichen Kupplung (110) gekoppelt wird.

7. Kupplungsanordnung nach Anspruch 6, wobei die erste Öffnung (205) in dem ersten Ende (255) der weiblichen Kupplung (110) verformbar ist, wenn der Abschnitt der männlichen Kupplung (115) derart in die erste Öffnung (205) eingeführt wird, dass der Abschnitt der männlichen Kupplung (115) in das Aufnahmeglied (225) eingeführt werden kann.

8. Kupplungsanordnung (105) nach Anspruch 7, wobei der erste Hauptkörper (200) der weiblichen Kupplung (110) ferner einen Flanschgliedhals (355) aufweist, wobei der Flanschgliedhals (355) derart ausgestaltet ist, dass er angeordnet wird, um mit dem ersten Flanschglied (520), das sich von der äußeren Oberfläche des zweiten Hauptkörpers (510) der männlichen Kupplung (115) weg erstreckt, in Eingriff zu gelangen, wenn der Abschnitt der männlichen Kupplung (115) in die erste Öffnung (205) der weiblichen Kupplung (110) eingeführt wird.

9. Kupplungsanordnung (105) nach Anspruch 8, wobei das erste Ende (255) der weiblichen Kupplung (110) verformbar ist, um zu ermöglichen, dass der Flanschgliedhals (355) mit dem ersten Flanschglied (520) außer Eingriff gelangt, um zu ermöglichen, den Abschnitt der männlichen Kupplung (115) aus der ersten Öffnung (205) der weiblichen Kupplung (110) zu entfernen.

10. Kupplungsanordnung (105) nach Anspruch 4, wobei die Daumenkissen (230) und das Dichtungsglied (330) während eines einzigen Umspritzungsprozesses geformt werden.

## Revendications

1. Ensemble de raccord à connexion/déconnexion rapide (105), comportant un raccord femelle (110) et un raccord mâle conjugué (115), le raccord femelle (110) comprenant :
un corps principal (200) définissant un passage de fluide (335) à travers lui ;
une première extrémité (255) définissant une ouverture (205) ;
un organe de réceptacle (225) positionné à l'intérieur du corps principal (200) autour du passage de fluide (335), l'organe de réceptacle (225) étant dimensionné pour recevoir une portion dudit raccord conjugué (115) qui peut être inséré à travers l'ouverture (205) dans la première extrémité (255) ; et
un organe de joint déformable (330) positionné à l'intérieur de l'organe de réceptacle (225), l'organe de joint (330) comportant une surface d'étanchéité assurant un joint étanche aux fluides avec le raccord conjugué (115), ledit organe de joint (330) comportant deux surfaces d'étanchéité (405, 410) comprenant une surface d'étanchéité primaire (405) positionnée de manière à s'engager avec une extrémité (535) du raccord conjugué (115), et une surface d'étanchéité secondaire (410) devant s'engager avec une surface latérale (530) du raccord conjugué (115) ;
l'ensemble de raccord (105) étant **caractérisé en ce que** ladite surface d'étanchéité secondaire (410) est positionnée dans une orientation perpendiculaire par rapport à la surface d'étanchéité primaire (405).

2. Ensemble de raccord (105) selon la revendication 1, dans lequel :
l'ouverture (205) dans la première extrémité (255) du corps principal (200) a une forme elliptique au repos ;
l'ouverture (205) dans la première extrémité (255) est déformable de telle sorte que la portion du raccord conjugué (115) puisse être insérée dans l'ouverture (205) ; et
le corps principal (200) du raccord femelle (110) comporte en outre un collier d'organe de bride (355), le collier d'organe de bride (355) étant configuré pour s'engager avec le raccord conjugué (115) lorsque la portion du raccord conjuguée (115) est insérée dans l'ouverture (205).

3. Ensemble de raccord (105) selon la revendication 2, dans lequel la première extrémité (255) est déformable de manière à désengager le collier d'organe de bride (355) du raccord conjuguée (115) afin de permettre au raccord conjugué (115) d'être retiré de l'ouverture (205) du raccord (110).

4. Ensemble de raccord (105) selon la revendication 3, le raccord femelle (110) comprenant en outre des plages pour le pouce opposées (230) positionnées autour du corps principal (200) pour permettre à un utilisateur de venir en contact avec les plages pour le pouce (230) et de déformer la première extrémité (255).

5. Ensemble de raccord (105) selon la revendication 1, dans lequel les surfaces d'étanchéité primaire et secondaire (405, 410) de l'organe de joint (330) sont formées au cours d'un processus de surmoulage unique.

6. Ensemble de raccord (105) selon l'une quelconque des revendications précédentes, **caractérisé par** :
un premier dispositif de raccord (100) selon l'une quelconque des revendications précédentes, et
un deuxième dispositif de raccord (115) prévu pour être inséré à travers la première ouverture (205) dans la première extrémité (255) ;
le raccord mâle (115) comportant :
un deuxième corps principal (510) incluant une surface extérieure (530) et définissant un deuxième passage de fluide (620) ;
une première extrémité (535) incluant une surface d'extrémité entourant une deuxième ouverture ; et
un premier organe de bride (520) s'étendant depuis la surface extérieure du deuxième corps principal (510) ;
la surface d'extrémité de la première extrémité (535) du raccord mâle (115) s'engageant avec la surface d'étanchéité primaire (405), et la surface d'étanchéité secondaire (410) s'engageant avec la surface extérieure (530) du corps principal (510) du raccord mâle (115) lorsque le raccord mâle (115) est accouplé au raccord femelle (110).

7. Ensemble de raccord selon la revendication 6, dans lequel la première ouverture (205) dans la première extrémité (255) du raccord femelle (110) est déformable à mesure que la portion du raccord mâle (115) est insérée dans la première ouverture (205) de telle sorte que la portion du raccord mâle (115) puisse être insérée dans l'organe de réceptacle (225).

8. Ensemble de raccord (105) selon la revendication 7, dans lequel le premier corps principal (200) du raccord femelle (110) comporte en outre un collier d'organe de bride (355), le collier d'organe de bride (355) étant configuré de manière à être positionné pour s'engager avec le premier organe de bride (520) s'étendant depuis la surface extérieure du deuxième corps principal (510) du raccord mâle (115) lorsque la portion du raccord mâle (115) est insérée dans la première ouverture (205) du raccord femelle (110).

9. Ensemble de raccord (105) selon la revendication 8, dans lequel la première extrémité (255) du raccord femelle (110) est déformable pour permettre au collier d'organe de bride (355) de se désengager du premier organe de bride (520) pour permettre à la portion du raccord mâle (115) d'être retirée de la première ouverture (205) du raccord femelle (110).

10. Ensemble de raccord (105) selon la revendication 4, dans lequel les plages pour le pouce (230) et l'organe de joint (330) sont formés au cours d'un processus de surmoulage unique.
